# EUROPEAN PATENT APPLICATION

(11) **EP 3 300 761 A1**
(43) Date of publication of application: **04.04.2018**
(21) Application number: 16306293.8
(22) Date of filing: 03.10.2016
(51) Int. Cl.: A61M 16/08, A61M 16/06

(54) **HOLLOW CONNECTOR WITH A GAS VENTING SYSTEM SUITABLE FOR RESPIRATORY MASK**

(71) Applicant: Air Liquide Medical Systems, 92160 Antony (FR)
(72) Inventor: ALBERICI, Luca, 25030 RONCADELLE (BS) (IT); MASSERDOTTI, Fulvio, 25075 BRESCIA (IT); FRANZONI, Mattia, 25021 Bagnolo Mella (Bs) (IT); BUGATTI, Ottorino, 25068 Sarezzo (Bs) (IT)
(74) Representative: Pittis, Olivier

(57) **Abstract**

A hollow connector (1) for respiratory mask (20) comprising a tubular body (2) traversed by an inner passage (3) comprising an inlet orifice (4) and an outlet orifice (5). A tubular sleeve (11) is arranged around the tubular body (2), said tubular sleeve (11) comprising a free open end (13) and a blind end (12), and delimits a venting passage (14) between the tubular sleeve (11) and the tubular body (2). The free open end (13) of the tubular sleeve (11) is arranged around the outlet orifice (5) of the tubular body (2). The tubular sleeve (11) is fixed, by the blind end (12), to the peripheral outer wall (6) of the tubular body (2). The blind end (12) of the tubular sleeve (11) further comprises a plurality of venting holes (10) putting the venting passage (14) in fluid communication with the atmosphere. Respiratory mask (20), such as a facial, comprising a mask body (21), a flexible cushion (23) and gas inlet (22) for providing air to the mask body (21), and a hollow connector (1) according to the invention fluidly connected to the gas inlet (22) of the mask body (21).

## Description

The invention concerns a hollow connector with improved venting ports used for venting CO₂-rich gases to the atmosphere, while the patient is expiring gases, and a respiratory mask, in particular a facial mask equipped with such a hollow connector, in particular for adult or pediatric uses in the treatment of respiratory conditions or diseases affecting adults, children, toddlers, infants or newborns.

Respiratory masks are commonly used for delivering non-invasive positive pressure ventilation (NPPV) or for continuous positive airway pressure (N-CPAP) therapy in disordered breathing conditions, such as obstructive sleep apnea (OSA), chronic obstructive pulmonary disease (COPD).

In both cases, the mask delivers a flow of breathable gas for or to assist in patient respiration. Actually, a mask typically comprises a rigid or semi-rigid hollow shell, usually made of polymer, defining a breathing chamber that receives at least a part of the patient's nose in case of a nasal mask, or both the nose and the mouth of the patient in case of a facial mask. The hollow shell or body receives the gas from a gas supply line, fixed to an inlet orifice arranged in the mask body by means of a hollow gas connector, for delivering the respiratory gas, such as air under pressure, into the breathing chamber of the shell.

A soft face-contacting cushion comes into contact with the patient's face and conforms to the various facial contours of the patient face thereby ensuring a gas tightness. The cushion is usually made of soft, resilient elastomeric material, such as soft silicone or similar.

The mask may also include a forehead support and further a headgear for correctly positioning, maintaining and/or securing the mask on the head of a patient.

Examples of masks are given by EP-A-462701, EP-A-462701, EP-A-874667, EP-A-1972357 and WO-A-00/57942.

During expiration phases, the CO₂-enriched gas flow exhaled by the patient should be vented to the atmosphere for avoiding or limit CO₂ re-inhalation by the patient during the inhalation phases. To this end, it is common to arrange one or several venting ports either in the mask shell, as for example taught by EP-A-1582230, or in the elbow portion of the tubular connector connected to the mask shell, as taught for example by WO-A-2005/051468, WO-A-2013170290 or EP-A-2281597.

Further, EP-A-2954919 and US-A-6584977 teach to arrange the venting holes in the annular border surrounding the inlet orifice of the mask body.

However, such arrangements of venting holes are either not always efficient and/or too noisy when expired-gases pass through them, while the patient is exhaling CO₂-rich gas, which creates a discomfort for the patient, especially when the patient has to wear the mask overnight, and also for the person, like spouse or husband, that sleeps next to the patient.

In other words, there is still a need for improving the venting to the atmosphere of the CO₂-enriched gas exhaled by the patient.

The problem to be solved is hence to improve the venting to the atmosphere of the CO₂-enriched gas exhaled by the patient.

In this goal, the present invention provides an improved hollow connector allowing an efficient CO₂-gas venting to the atmosphere as well as a respiratory mask, especially a facial mask recovering the nose and mouth of a patient, equipped with such an improved hollow connector.

More precisely, the solution of the present invention concerns a hollow connector, i.e. a gas connector, for a respiratory mask, especially a facial mask, comprising a tubular body traversed by an inner passage, in which a respiratory gas can circulates, comprising an inlet orifice for receiving the gas and an outlet orifice for delivering the gas, characterized in that:
- a tubular sleeve is arranged around the tubular body, said tubular sleeve comprising a free open end and a blind end, and delimits a venting passage between the tubular sleeve and the tubular body,
- the free open end of the tubular sleeve is arranged around the outlet orifice of the tubular body,
- the tubular sleeve is fixed, by the blind end, to the peripheral outer wall of the tubular body, and
- the blind end of the tubular sleeve further comprises a plurality of venting holes putting the venting passage in fluid communication with the atmosphere, i.e. ambient air surrounding the connector.

The hollow connector according to the present invention can further comprise one or more of the following additional features:
- it is designed and adapted for conveying a gas, such as air under pressure (i.e. > 1 bar).
- the venting ports are orifices that allow the venting of gas to the atmosphere, i.e. to the surrounding ambient air.
- at least part of the venting ports have a generally tronconical shape, i.e. their inner diameters situated in the bottom or blind end of the tubular sleeve, are greater that their outer diameters.
- the tubular body comprises an annular shoulder integral with, i.e. firmly fixed to, the peripheral outer wall of the tubular body, said annular shoulder projecting away from said peripheral outer wall and further comprising the blind end of the tubular sleeve and the plurality of venting holes.
- the tubular sleeve is a tube element coaxially arranged around the tubular body of the connector.
- the plurality of venting holes are arranged in arc of circle.
- alternatively, the venting ports are distributed all along the circumference.
- the plurality of venting holes are arranged in a unique row.
- alternatively, the venting ports are arranged in several rows, for instance 2 or 3 rows.
- the plurality of venting holes comprises at least 5 holes, preferably at least 10 holes.
- the holes have a inlet and an outlet diameters of between about 0,3 mm and 3 mm, the inlet diameter being greater than or equal to the outlet diameter.
- the different holes can have the same or different inlet an outlet diameters.
- the tubular body has a generally curved shaped, i.e. an elbow shape or an "L" shape.
- the tubular body has a generally curved shaped comprising an elbow portion and straight or linear portions/segments sandwiching the elbow portion/segment.
- the tubular sleeve and the tubular body are preferably molded in one piece.
- alternatively, the tubular sleeve and the tubular body can be made of several components firmly fixed together, for example screwed, snap-fitted and/or glued together.
- the tubular sleeve and the tubular body are preferably made of a plastic material or similar, such as polycarbonate (PC), polypropylene (PP) or nylon.
- the hollow connector has an inner diameter of the inner gas passage of between about 1 and 3 cm, measured at the outlet orifice.
- it further comprises an anti-asphyxia system (AAS) comprising a venting port cooperating with a mobile flap. Such an AAS is known from US-A-5438981 and WO-A-00/38772. Such AAS are useful in facial masks in case of failure of the gas delivery apparatus.
- the venting holes have their axis parallel or titled relative to the axis of the tubular sleeve.

The invention also concerns a respiratory mask, especially a facial mask, comprising a mask body comprising a gas inlet, and a hollow connector according to the present invention, i.e. described hereabove and hereafter, fluidly connected to the gas inlet of the mask body.

The mask according to the present invention can further comprise one or more of the following additional features:
- it comprises a hollow body, also called mask shell or mask body, comprising an inner chamber and a gas inlet in fluid communication with said inner chamber.
- the gas inlet of the mask hollow body is configured for fixing thereto a tubular gas connector according to the present invention.
- the gas inlet orifice of the mask hollow body has a diameter of between about 1 and about 3 cm.
- the gas inlet orifice is arranged substantially centrally in the mask body.
- when the hollow connector according to the present invention is fluidly connected to the mask hollow body, the inner chamber of the mask body is in fluid communication with the ambient atmosphere through the venting passage and the venting ports of the hollow tubular connector according to the present invention so as to allow venting the CO₂-expired gas to be vented to the atmosphere by the venting ports, during expiration phases of the patient.
- the tubular connector comprises a front tubular portion or segment cooperating with the mask body so as to maintain the tubular gas connector integral with the mask body.
- the tubular connector comprises a rear tubular portion or segment for receiving and securing thereto a hose or gas line by means of a connecting piece, such as intermediary connector that is rotatable with respect to the tubular connector of the invention, i.e. the elbow-shape tubular connector of the invention.
- when the tubular hollow connector of the invention is inserted into the gas inlet of the mask body, the gas passage traversing the hollow connector is in fluid communication with the inner chamber of the mask body for delivering respiratory gas therein, whereas said inner chamber of the mask body is further in fluid communication with the venting passage and with the venting holes of the hollow connector of the invention for venting expired-gases to the ambient atmosphere, i.e. out of the mask's inner chamber.
- it further comprises a flexible cushion fixed to the mask body, for instance the cushion can be snap-fitted to the mask body, or fixed thereto by means other means, such as glued to it or even molded in one-piece.
- the cushion can be either a nasal cushion receiving only the nose of the patient (i.e. in the case of a nasal mask), or a facial cushion receiving both the nose and the mouth of the patient (i.e. in the case of a facial mask).
- the mask is preferably a facial mask covering, in use, the nose and the mouth of the patient.
- preferably, the cushion is a facial cushion configured and sized for coming into contact, when the mask is worn by a user, i.e. a patient, with specific regions of the user's face, comprising a nasal bridge region including lateral nose regions, a chin region, i.e. the area located under the lower lip, and cheek regions located on each lateral sizes of the nose and mouth, said cheek regions linking the nasal bridge region including lateral nose regions to the chin region. Actually, the nasal bridge region can be an area of the nose located either at the junction of bone and cartilage, or below or above said bone/cartilage junction of the nose; the chin region is expending from the lower lip to the chin, whereas the cheek regions are the areas of the face located on right and left sizes of the nose and mouth.
- the cushion comprises a cushion body delimiting a respiratory chamber comprising a front aperture (i.e. front opening) adapted for receiving at least part of the patient's nose and/or mouth, in use, i.e., when the patient wears the mask, especially a facial mask.
- the cushion comprises a cushion body delimiting a respiratory chamber further comprising a rear aperture (i.e. rear opening) that opens into the inner chamber of the mask body, when the cushion is firmly fixed to the mask body, so that the respiratory chamber of the cushion is in fluid communication with the inner chamber of the mask body.
- the cushion has a generally tridimensional (3D) shape, preferably a 3D saddle, triangular or trapezoidal shape.
- the front aperture (i.e. front opening) of the cushion is configured and sized for receiving at least part of the patient's nose and/or mouth, in use.
- the cushion comprises at least one membrane forming a kind of flexible skirt along the cushion aperture receiving the patient's face, i.e. nose and/or mouth.
- the cushion comprises a membrane forming a flexible skirt along the cushion aperture that deforms so as to match the contours of the face of the patient thereby ensuring gas tightness when the patient inserts his/her face, i.e. nose or nose and mouth, into the front aperture of said cushion.
- in another embodiment, the flexible cushion can comprise several membranes, e.g. two or three superimposed membranes that ensure gas tightness, the outer membrane being in contact with the patient's face, when the patient wears the mask.
- the one or several membranes are arranged around all along the periphery of the front aperture of the respiratory chamber of the cushion.
- the cushion is made of a soft flexible material, preferably silicone or similar.
- it further may comprise a front element, also called "shroud", "retainer" or "headgear-receiving structure", arranged between the hollow connector and the mask body, said front element comprising headgear connecting structures.
- alternatively, the headgear connecting structures can be carried directly by the mask body, i.e. in the case where there is no front "shroud" or "retainer".
- the headgear connecting structures comprise hooks, slots, snap-fit connectors or similar, including combination thereof.
- it may further comprise a holding arm projecting upwardly from the hollow body and/or two lateral arms projecting laterally from the hollow body.
- alternatively, the front shroud comprises a holding arm projecting upwardly, i.e. in use, in front of the nose and of part of the forehead of the patient, and/or two lateral arms projecting laterally, i.e. in use, along part of the cheeks of the patient.
- preferably, the two lateral arms projecting laterally and/or the holding arm projecting upwardly are integrally fixed to the hollow body or to the front shroud, for instance molded in one-piece or glued together.
- the holding arm and/or of the two lateral arms are made of a flexible material, such as a polymer material, e.g. polycarbonate (PC), polypropylene (PP) or nylon, so as to be slightly bendable toward the patient's face under the forces applied by the headgear, when said headgear is adjusted to the morphology of the patient's head.
- a headgear is fixed to the headgear connecting structures.
- the two lateral arms and/or the holding arm each comprises a free distal end carrying the headgear connecting structures.
- a headgear comprising several straps is fixed to the headgear connecting structures of the two lateral arms and/or of the holding arm, preferably the straps are made of fabric or polymer materials or the like.
- the holding arm and/or of the two lateral arms have an elongated shape, for example a band or ribbon shape. Of course, other shapes are possible.
- the holding arm has a length of about 2 and 12 cm.
- each lateral arms has a length of about 3 and 8 cm.
- when carried by the front shroud, at least a region of the holding arm and/or of the two lateral arms is configured or shaped so as to spouse, i.e. to match, the external profile, i.e. the outer contours, of the mask body, when the front shroud is positioned in contact of the mask body. Preferably, They have a curved-shape.
- the hollow connector, preferably a curved connector, fixed is to the front side of the body mask so as to be in fluid communication with one another thereby allowing gas flows to circulate, in normal use:
   - from the hollow connector of the invention to the inner chamber of the mask body during the inspiration phases of the patient, when air under pressure is delivered to the patient,
   - and vice versa, i.e. from the mask body to the hollow connector of the invention, during expiration phases, when the patient exhales CO₂-enriched gases that have to be vented to the atmosphere through the venting passage and venting holes of the tubular sleeve arranged around the tubular body of the hollow connector of the invention.
- the hollow connector is fixed to and rotatable with respect to the mask body.
- the hollow connector is fixed to the mask body by connecting structures comprising male/female connection elements, such as grooves, walls, abutments... or any other kind of connecting system, for instance by a snap-fit connection or the like.

The invention also concerns an assembly for delivering a gas to a patient comprising a gas delivery device, such as a medical ventilator, i.e. a respiratory assistance apparatus, and a respiratory mask with a hollow curved connector according to the present invention.

Preferably, the gas delivery device is fluidly connected to the hollow connector and the respiratory mask by means of a gas line, such as a flexible hose, said gas line being fluidly connected to the hollow connector of the present invention by means of a intermediary connector that is rotatable with respect to the hollow curved connector according to the present invention.

An embodiment of a facial respiratory mask according to the present invention is shown in the enclosed illustrative, but not limitative, Figures, among which:
- Figure 1 represents a general view of an embodiment of a facial mask equipped with a hollow connector according to the present invention,
- Figure 2 is an exploded view of the facial mask of Figure 1,
- Figure 3 is an enlarged and partial front view of a mask according to Figures 1 and 2,
- Figure 4 is an enlarged and partial rear view of a mask according to Figures 1 and 2,
- Figures 5 is front view of an embodiment of a hollow curved connector according to the present invention,
- Figures 6 and 7 are sagittal views of a hollow curved connector according to the present invention.

The present invention proposes a new structure of a hollow gas connector 1, preferably a curved connector, with venting ports 10 for a respiratory mask as shown in Figures that illustrate some embodiments of a facial mask 20 and a curved gas connector 1 according to the present invention.

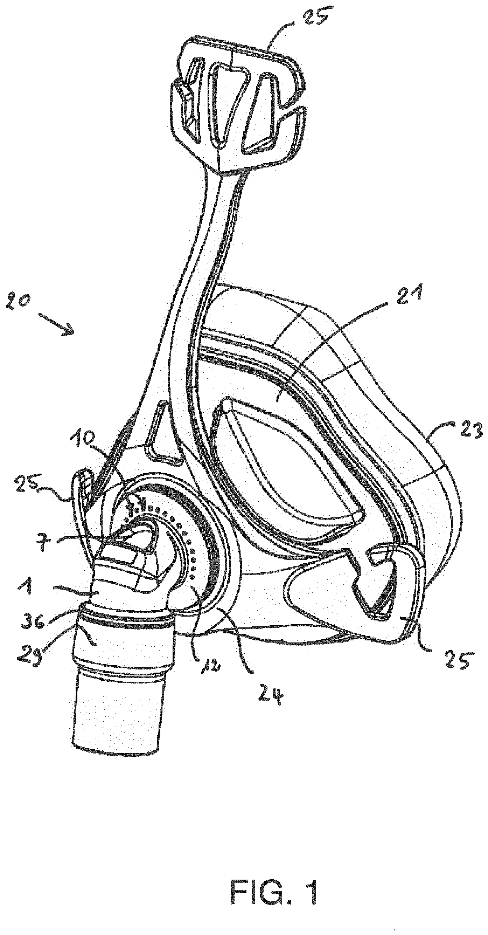

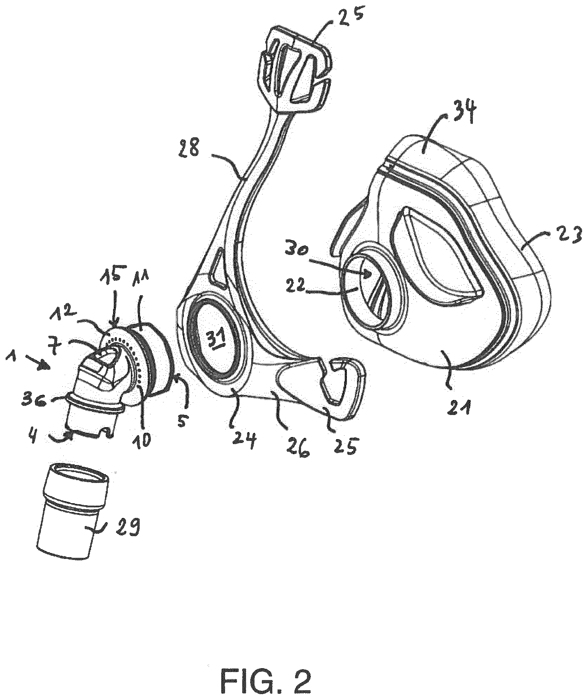

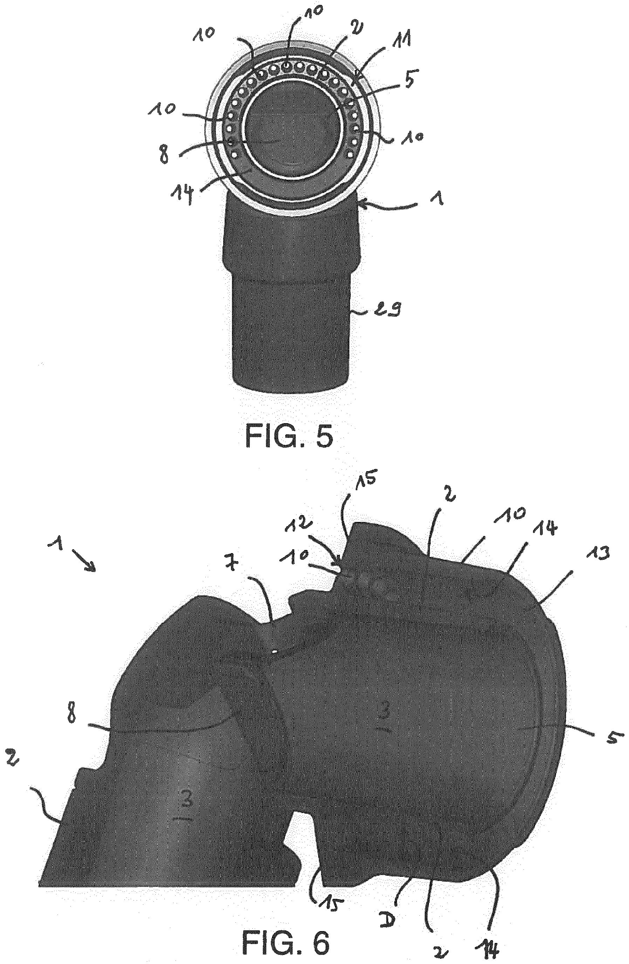

Figures 1 and 2 represent a general view of an embodiment of a respiratory mask 20, namely here a facial mask, according to the present invention, comprising a hollow curved connector 1 according to the present invention including a plurality of venting holes 10 that are located in the bottom 12 of a blind sleeve 11 arranged around one of the free ends of the connector body 2, as detailed on Figures 5 and 6, and further described hereafter.

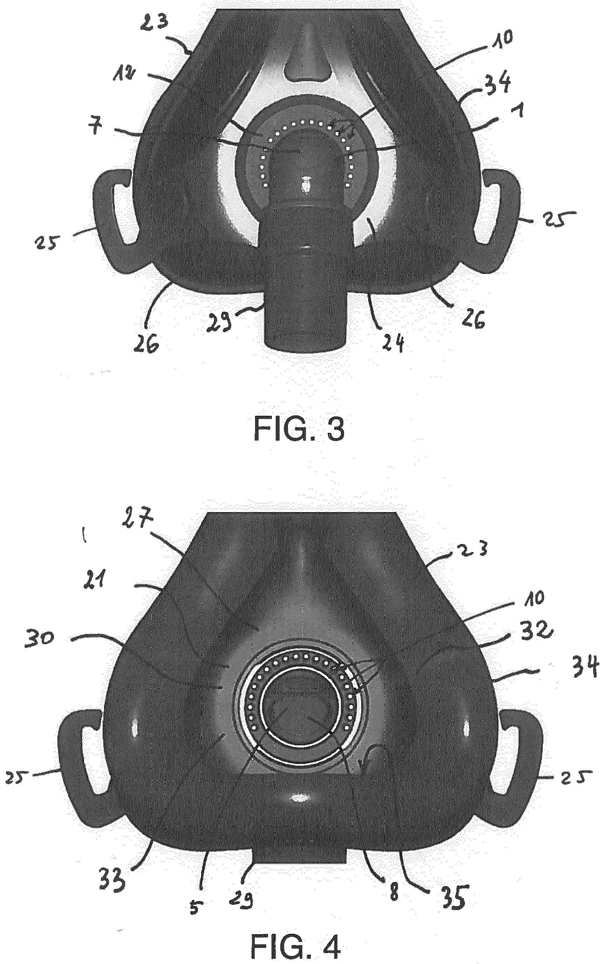

The mask 20 comprises a mask body or shell 1 defining an inner chamber 30 comprising a gas inlet 22 in fluid communication with said inner chamber 30, as visible in Figures 2 and 4.

The gas inlet 22 is designed for fixing thereto a tubular gas connector 1 according to the present invention, as detailed hereafter.

In the embodiment presented in Figures 1 and 2, a front element or shroud 24 is "sandwiched" between the tubular gas connector 1 and the mask body 21 of the mask 20.

The front shroud 24 is carrying two lateral arms 26 and a holding arm 28 that are integrally fixed to or molded in one-piece with the rest of the shroud 24.

When the front element 24, i.e. shroud or retainer 24, is fixed to the mask body 21, the holding arm 28 projects upwardly, i.e. about vertically, with respect to the mask body 21, whereas the two lateral arms 26 projecting laterally from said mask body 21 in opposite directions, i.e. one toward the right side of the mask 20 and the other toward the left side of the mask 20, as shown in Figures 1 and 2.

The two lateral arms 26 and the holding arm 28 have free distal ends carrying headgear connecting structures 25, such as slots, hooks or the like, so as to fix a headgear thereto. A headgear typically comprises several straps of fabric or polymer materials or the like, and is used for securing and maintaining the mask 20 in position on the patient's face, when the mask is used, i.e. worn by the patient.

The holding arm 28 and/or of the two lateral arms 26 have typically elongated shapes, for example band or ribbon shapes, and are preferably made of a flexible material, such as a polymer material, e.g. polycarbonate (PC), polypropylene (PP) or nylon so as to be slightly bendable toward the patient's face under the forces applied by the headgear, when said headgear is adjusted to the morphology of the patient's head. The front shroud 24 can be made of the same polymer material or of any other suitable material.

As visible on Figure 2, the shroud 24 further comprises a central aperture 31 that is traversed by part of the hollow connector 1 and/or of the mask body 21 as can be derived from Figures 1 and 2.

However, it should be emphasized that the front element or shroud 24 is only an optional element and depending on the embodiment, it can be ignored/omitted. Thus, in an alternative embodiment (not shown), the front shroud 24 does not exist as the holding arm 28 and/or of the two lateral arms 26 are directly fixed to the mask body 21.

Furthermore, the mask body 21 can be made from a unique piece or from several pieces or sub-units that are subsequently assembled together and made integral by means of any available fixing technique, for instance they can be glued, thermo-welded or similarly affixed together. They can be made of a polymer material that is slightly flexible, preferably a polymer or plastic material, such as polypropylene (PP), polycarbonate (PC) and nylon, or similar.

Further, the facial mask 20 of the present invention also comprises a flexible cushion 23 that is fixed to the rear side of the mask body 21. Typically, the flexible cushion 23 is a tridimensional hollow structure forming a respiratory chamber 27 with an aperture adapted, i.e. conformed and sized, for receiving at least part of the nose and mouth of the patient as above explained, when the patient wears the mask, so that the patient can breathe the respiratory gas contained in the inner chamber 30 of the mask body 21 and/or in the respiratory chamber 27 of the cushion 23 as those chambers 30, 27 are in fluid communication with one another.

Indeed, the cushion 23 comprises a cushion body 34 delimiting the respiratory chamber 27 and further comprises a rear aperture, i.e. a rear opening that opens into the inner chamber 30 of the mask body 21, when the cushion 23 is firmly fixed to the mask body 21, so that gas can freely pass from the inner chamber 30 of the mask body 21 towards the respiratory chamber 27 of the cushion 23, and vice versa.

Actually, the inner chamber 30 of the mask body 21 and the respiratory chamber 27 of the cushion 23 form together a large compartment wherein the patient can inspire fresh gas, such as air, during inhalation phases, and exhale CO₂-enriched gas, during expiration phases.

In other words, the respiratory chamber 27 of the cushion 23 and the inner chamber 30 of the mask body 21 are both in fluid communication with the central gas passage 3, i.e. the lumen, of the elbow connector 1 of the invention so that a gas flow can freely travel from said tubular connector 1 to said chambers 27, 30, or vice versa, as detailed below.

In order to provide efficient gas tightness (i.e. seal) and/or increased comfort for the patient, the aperture of the cushion 23 is delimited by a flexible membrane 32 that comes into contact with the patient's face and spouses his/her facial morphology, when the mask 20 is worn by the patient. This membrane 32 constitutes a kind of soft flexible skirt delimiting the aperture of the cushion 23.

Of course, depending on the embodiment, two or more superimposed membranes 32 can also be used as using several membranes may improve the gas tightness in certain circumstances, e.g. for some particular patient's morphologies.

The cushion 23 has, in the present case, a generally tridimensional (3D) trapezoidal shape, but other shapes are also possible such as triangular or saddle shapes, so as to better fit with the contours of the patient's face, especially in the nasal regions.

When the facial mask 20 is worn by the patient, i.e. when the front aperture 33 of cushion 23 receives at least a part of the nose and the mouth of the patient, i.e., when the patient introduces his/her nose into the internal volume defined by the inner and the respiratory chambers 27, 30, and breathes the gas contained therein or exhales CO₂-enriched gases into it, the cushion 23 and the membrane 32 forming the peripheral border 35 of the front aperture 33 of the cushion 23 are in contact with particular and well defined regions of the patient's face, thereby ensuring gas tightness.

Preferably, the cushion 23 is a facial cushion configured and sized for coming into contact, when the mask is worn by a user, with at least the nasal bridge region (including lateral nose regions), the chin region, i.e. the area located under the lower lip, and the cheek regions located on both lateral sizes of the nose and mouth of the patient, said cheek regions linking the nasal bridge region including lateral nose regions to the chin region. Actually, the nasal bridge region can be an area of the nose located either at the junction of bone and cartilage, or below or above said bone/cartilage junction of the nose; the chin region is expending from the lower lip to the chin, whereas the cheek regions are the opposite areas located on right and left sizes of the nose and mouth.

Of course, other embodiments and shapes of the cushion 23 are possible.

The cushion 23 (i.e., including cushion body 34 and membrane 32) is preferably made of a flexible and soft material, such as silicone or the like. Preferably, the membrane 32 and the cushion body 34 are molded in one piece.

The cushion 23 is further fixed to the mask body 21 by means of a connecting system, such as male/female connections, that can comprise peripheral groove(s) and wall(s) arranged on the cushion 23 cooperating with complementary peripheral wall(s) and/or groove(s) arranged on the body 21 of the mask 20 that are configured or shaped so as to perfectly fit together, for ensuring for instance a snap-fit connection or the like. Depending on the embodiment, the connecting system can also comprise threaded structures cooperating together, a bayonet fixing mechanism or any other fixation system.

The main improvement according to the present invention concerns the venting ports 10 that are used for venting expired CO₂-rich gases to the atmosphere through those venting ports 10, when the patient exhales.

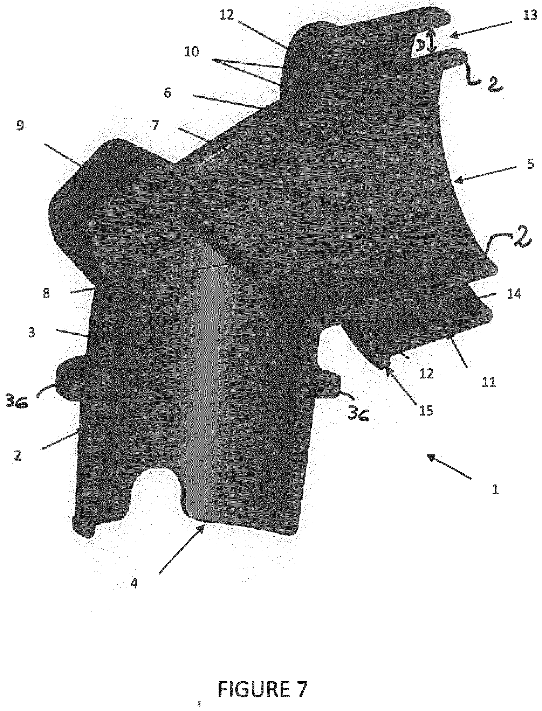

Indeed, according to the present invention, several venting ports 10 are arranged in a particular structure of the hollow connector 1 as clearly illustrated in figures 6 and 7.

The particular structure of the hollow connector 1 according to the present invention will be detailed now.

As shown in Fig. 6 and 7, the hollow connector 1 of the invention, that is useable for use with a respiratory mask 20 as illustrated in Figures 1 and 2 for instance, has a tubular body 2 traversed by an inner passage 3 comprising an inlet orifice 4 and an outlet orifice 5.

The tubular body 2 is preferably curved so as to have a L- or elbow-shape. In other words, tubular body 2 comprises an upstream linear (i.e. straight) portion comprising the inlet orifice 4, a downstream linear (i.e. straight) portion or segment comprising the outlet orifice 5 and an intermediary curved portion or segment comprising an elbow or the like, said intermediary curved portion or segment being sandwiched between the two linear portions or segments.

The inner passage 3 passes through the two linear portions and the intermediary curved portion thereby linking the inlet orifice 4 and the outlet orifice 5 so as to be able to convey fresh gas, such as pressurized air, from the inlet orifice 4 to the outlet orifice 5. The inner passage 3 has generally a circular cross section.

As one can see in Fig. 1-3, the upstream portion of the tubular body 2 of the hollow connector 1, that comprises the inlet orifice 4, is fed with gas, such as pressurized air, by a flexible hose (not shown) or the like conveying the gas delivered by a medical gas source, such as a medical ventilator or apparatus. The flexible hose is fluidly connected to the tubular body 2 of the hollow connector 1 of the invention by means of an intermediary connector 29, i.e. a tubing element or a tubular piece, that is connected, on the one hand, to the upstream portion of the tubular body 2 of the hollow connector 1, for instance connected by a snap-fit system, and, on the other hand, to the flexible hose conveying the respiratory gas.

For limiting the course of the intermediary connector 29, while it is connected, i.e. snap-fitted or similar, to the upstream portion of the tubular body 2 of the hollow connector 1, a annular abutment 36 is provided around the upstream portion of the tubular body 2, which forms an annular border or ring, as shown in Fig. 1, 2 and 7.

Further, the intermediary connector 29 has generally tubular shape and is made of a polymer material, i.e. plastic material. It is further coaxially arranged to the upstream portion of the tubular body 2 of the hollow connector 1 and is preferably rotatable with respect to the hollow curved connector 1 according to the present invention.

According to the present invention, the tubular body 2 further comprises a tubular sleeve 11, i.e. a tube (or pipe) element or the like, that is arranged around the tubular body 2, namely around the downstream linear portion comprising the outlet orifice 5. Preferably, the tube element 11 is coaxially arranged around the tubular body 2 of the hollow connector 1.

The tubular sleeve 11 comprises a free open end 13 and a blind end 12, and delimits a venting passage 14 between the tubular sleeve 11 and the tubular body 2.

As shown in Fig. 6 and 7, the free open end 13 of the tubular sleeve 11 is arranged around the outlet orifice 5 of the tubular body 2, i.e. the free open end 13 of the tubular sleeve 11 and the outlet orifice 5 of the tubular body 2 are coaxially arranged.

Further, the tubular sleeve 11 is arranged at a distance D of several millimeters from the peripheral outer wall 6, i.e. the outer surface, of the tubular body 2 (pref. 30 mm > D > 0) so as to create or delimit between them a venting passage 14 for recovering CO₂-riched expired gases exhaled by the patient. The venting passage 14 has hence an annular cross-section.

The exhaled gas enters into the venting passage 14 by the free open end 13 of the tubular sleeve 11 and travels therein toward venting orifices 10, as explained below.

The tubular sleeve 11 further comprises a blind end 12 that is located opposite to the free end 23 and that is fixed to the peripheral outer wall 6 of the tubular body 2, thereby forming a bottom or closing wall of the tubular sleeve 11

A plurality of venting holes 10 are arranged in the blind end 12 of the tubular sleeve 11. Those venting holes 10 fluidly communicate, on the one hand, with the venting passage 14 delimited by the inner surface of the tubular sleeve 11 and the outer surface, i.e. the peripheral outer wall 6, of the tubular body 2, and, on the other hand, with the ambient atmosphere, i.e. ambient air, surrounding the connector 1. In other words, the plurality of venting holes 10 puts the venting passage 14 in fluid communication with the ambient atmosphere so as to wash out expired-gas present into the venting passage 14.

Actually, according to the present invention, when the patient expires CO₂-enriched gas, during the expiration phases, at least a part of the expired gas first exits the mask body 21, then enters into the venting passage 14 of the connector 1 by the free end 13 of the sleeve 12, travels into said venting passage 14 towards the plurality of venting holes 10, and is eventually vented to the atmosphere, while passing through said venting holes 10. Preferably, the venting holes 10 have a (tron)conical shape, i.e. an enlarged section (i.e., diameter) on their inlet side than on their outlet side so as to facilitate the gas venting.

The venting holes 10 can have their axis parallel to the axis of the tubular sleeve 11and/or of the tubular body 2. In another preferred embodiment, the venting holes 10 have their axis oriented non-parallel, i.e. inclined, directed or titled toward the exterior of, i.e. away from, the connector 1, so as to provide a greater/better venting of the CO₂-enriched gas flow away from the connector 1 and improving the overnight comfort of a person, e.g. spouse or husband, sleeping with the patient treated for sleep apnea or any other respiratory disease.

In the embodiment of Figures 6 and 7, the tubular body 2 comprises an annular shoulder 15 integral with the peripheral outer wall 6 of the tubular body 2, and which projects away from said peripheral outer wall 6. In other words, the annular shoulder 15 defines a ring-like structure around the peripheral outer wall 6 of the tubular body 2.

The tubular sleeve 11 is attached to that annular shoulder 15 so that the tubular sleeve 11 constitutes the blind end 12 of the tubular sleeve 11, i.e. the closed bottom 12 of the tubular sleeve 11.

The venting holes 10 are hence arranged (e.g. bored) through said annular shoulder 15 as shown in Fig. 6 and 7.

The venting holes 10 can be arranged in an array forming a circle or preferably an arc of circle (i.e., semi-circular array) in the blind end 12 of the tubular sleeve 11 as shown in Fig. 5. In other words, the venting ports 5 are distributed all along the circumference of the closed bottom or blind end 12 of the tubular sleeve 11 or preferably along only a part of that circumference as represented in Fig. 1, 2, 4 and 5.

Of course, holes 10 can also be arranged in several arrays, e.g. coaxially arranged arrays, or in any other way.

Preferably, at least 5 venting holes are provided, more preferably at least 10 venting holes 10, advantageously between 12 and 30 venting holes 10.

Furthermore, the different elements of the connector 1, especially the tubular sleeve 11 and the tubular body 2 are preferably molded in one piece ; however, they can also be made of several elements or pieces, i.e. sub-units, that are fixed together, e.g., glued or the like.

Preferably, the connector 1 including the tubular sleeve 11 and the tubular body 2 is entirely made of a plastic material, such as polycarbonate (PC), polypropylene (PP) or nylon.

In the case of a facial mask, it is wise that the connector 1 further comprises an anti-asphyxia system 7, 8 comprising a venting port 7 cooperating with a mobile flap 8 so as to allow the patient breathing fresh air through the venting port 7 in case of failure of the gas source, such as a medical ventilator. Such asphyxia system 7, 8 are known in the art, as disclosed by US5438981 or WO-A-00/38772.

Generally speaking, the respiratory mask 20 of the present invention, such as a facial mask, is light, comfortable to wear, easy to position and secure on the patient's face, provide a good tightness (seal) and an efficient CO₂-enriched gas venting to the atmosphere thanks to the hollow connector according to the present invention.

The respiratory mask 20 of the present invention can be used in a method for treatment of a respiratory disorder or condition affecting infant, toddler and child patients as well as adult patients, for example in non-invasive positive pressure ventilation (NPPV) or in a nasal continuous positive airway pressure (N-CPAP) therapy of sleep disordered breathing (SDB) conditions, such as, for example, obstructive sleep apnea (OSA).

## Claims

1. Hollow connector (1) for respiratory mask (20) comprising a tubular body (2) traversed by an inner passage (3) comprising an inlet orifice (4) and an outlet orifice (5), **characterized in that**:
- a tubular sleeve (11) is arranged around the tubular body (2), said tubular sleeve (11) comprising a free open end (13) and a blind end (12), and delimits a venting passage (14) between the tubular sleeve (11) and the tubular body (2),
- the free open end (13) of the tubular sleeve (11) is arranged around the outlet orifice (5) of the tubular body (2),
- the tubular sleeve (11) is fixed, by the blind end (12), to the peripheral outer wall (6) of the tubular body (2), and
- the blind end (12) of the tubular sleeve (11) further comprises a plurality of venting holes (10) putting the venting passage (14) in fluid communication with the atmosphere.

2. Hollow connector according to the preceding Claim, **characterized in that** the tubular body (2) comprises an annular shoulder (15) integral with the peripheral outer wall (6) of the tubular body (2), said annular shoulder (15) projecting away from said peripheral outer wall (6) and further comprising the blind end (12) of the tubular sleeve (11) and the plurality of venting holes (10).

3. Hollow connector according to anyone of the preceding Claims, **characterized in that** the plurality of venting holes (10) are arranged in arc of circle.

4. Hollow connector according to anyone of the preceding Claims, **characterized in that** the tubular body (2) has a generally curved shaped.

5. Hollow connector according to anyone of the preceding Claims, **characterized in that** the tubular sleeve (11) and the tubular body (2) are molded in one piece.

6. Hollow connector according to anyone of the preceding Claims, **characterized in that** the venting holes (10) have a tronconical shape.

7. Hollow connector according to anyone of the preceding Claims, **characterized in that** the tubular sleeve (11) is a tube element coaxially arranged around the tubular body (2) of the connector (1).

8. Hollow connector according to anyone of the preceding Claims, **characterized in that** the venting holes (10) have their axis parallel to or titled relative to the axis of the tubular sleeve (11).

9. Hollow connector according to anyone of the preceding Claims, **characterized in that** the tubular sleeve (11) and the tubular body (2) are made of plastic material.

10. Hollow connector according to anyone of the preceding Claims, **characterized in that** it further comprises an anti-asphyxia system (7, 8) comprising a venting port (7) cooperating with a mobile flap (8).

11. Respiratory mask (20) comprising a mask body (21) comprising a gas inlet (22), and a hollow connector (1) according to anyone of the preceding Claims fluidly connected to the gas inlet (22) of the mask body (21).

12. Respiratory mask according to Claim 10, **characterized in that** it is a facial mask.

13. Respiratory mask according to Claim 10 or 11, **characterized in that** it further comprises a cushion (23) fixed to the mask body (21), preferably a cushion (23) made of silicone.

14. Respiratory mask according to anyone of Claims 10 to 13, **characterized in that** it further comprises a front element (24) arranged between the hollow connector (1) and the mask body (21), said front element (24) comprising headgear connecting structures (25).

15. Respiratory mask according to anyone of Claims 10 to 15, **characterized in that** it further comprises a headgear is fixed to the headgear connecting structures (25) of the front element (24).
